# EUROPEAN PATENT APPLICATION

(11) **EP 3 967 157 A1**
(43) Date of publication of application: **16.03.2022**
(21) Application number: 20195386.6
(22) Date of filing: 09.09.2020
(51) Int. Cl.: A23L 33/16, A23P 10/30, A61K 9/50, A23L 33/10, A61K 33/06

(54) **PLANT-BASED MICROCAPSULES**

(71) Applicant: Xampla Limited, Cambridge, Cambridgeshire CB4 0FW (GB); The Chancellor, Masters and Scholars of the University of Cambridge, Cambridge CB2 1TN (GB)
(72) Inventor: RODRIGUEZ GARCIA, Marc, Cambridge, Cambridgeshire CB4 0FW (GB); KNOWLES, Tuomas Pertti Jonathan, Cambridge, Cambridgeshire CB4 0FW (GB)
(74) Representative: Gurney, Steven

(57) **Abstract**

The present invention relates to plant-based microcapsules for the efficient encapsulation and retention of water-soluble ingredients, as well as the efficient co-encapsulation of water-soluble and water-insoluble ingredients. The present invention also relates to compositions comprising the microcapsules, a method of making the microcapsules and compositions, and to uses of the microcapsules and compositions.

## Description

### FIELD OF THE INVENTION

The present invention relates to plant-based microcapsules for the efficient encapsulation and retention of water-soluble ingredients, as well as the efficient co-encapsulation of water-soluble and water-insoluble ingredients. The present invention also relates to compositions comprising the microcapsules, a method of making the microcapsules and compositions, and to uses of the microcapsules and compositions.

### BACKGROUND

The development of biocompatible materials has attracted much interest in recent years. The demand for biocompatible materials with functional characteristics is particularly significant in applications where such materials come into contact with the human body, including in cosmetics, food and pharmaceuticals. However, the stability and functionality of a wide range of active compounds is limited in the bulk solution, where degradation and loss of function occur rapidly. In this context, the main advantages of encapsulation are in protecting sensitive or unstable compounds from degradation under adverse conditions, such as exposure to chemicals, air and light, and to allow control of the bio-accessibility and bioavailability of the encapsulated compounds. While several encapsulation techniques are currently available, these commonly rely on the use of synthetic polymers, which may not be suitable as carriers in pharmaceutical and food applications or may have limited permitted exposure levels in cosmetic applications. Furthermore, synthetic polymer shell materials can lead to the formation of microplastics, which are detrimental to the environment.

Alternatively, naturally-derived polymers such as chitosan or gelatine can be used in encapsulation techniques but the stability of such naturally-derived shell materials can be severely affected by processing conditions normally present during final product manufacturing processes (such as exposure to high temperatures and high shear conditions) or by the food or pharmaceutical product composition (pH, presence of chelating agents, cross-interaction with other ingredients etc.). To overcome this, covalent cross-linking strategies are often employed, which may lead to the presence of harmful unreacted cross-linking agents in the final product.

Furthermore, whilst the encapsulation of oils, or oil-soluble ingredients can be achieved using standard encapsulation approaches, the inherent shell permeability of microcapsules prepared from naturally-derived polymers does not allow for the efficient encapsulation of small water-soluble compounds and their retention within the microcapsules, particularly in an aqueous environment. It thus remains a challenge to generate a microencapsulation system from a naturally-derived polymer that can efficiently encapsulate small water-soluble ingredients, as well as withstand standard industrial manufacturing processing conditions without compromising the capsule stability.

### SUMMARY OF THE INVENTION

Viewed from a first aspect, the present invention provides a microcapsule comprising:
(a) a hydrophilic phase comprising a water-soluble ingredient;
(b) a lipophilic phase; and
(c) a plant-based protein hydrogel shell.

Viewed from a further aspect, the present invention provides a composition comprising at least one microcapsule as hereinbefore described and an external phase.

Viewed from a further aspect, the present invention provides a method for preparing a microcapsule as hereinbefore described, comprising:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein solution, to give a double emulsion;
(c) re-emulsifying said double emulsion in a second lipophilic phase to give a triple emulsion;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase; and
(e) washing the microcapsule to remove the second lipophilic phase.

Viewed from a further aspect, the present invention provides a microcapsule prepared according to the method as hereinbefore described.

Viewed from a further aspect, the present invention provides a method for preparing a composition as hereinbefore described, comprising:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein, to give a double emulsion;
(c) re-emulsifying said double emulsion in a second lipophilic phase to give a triple emulsion;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase.

Viewed from a further aspect, the present invention provides a composition prepared according to the method as hereinbefore described.

Viewed from a further aspect, the present invention provides a food, beverage, cosmetic, pharmaceutical, medical device, biomaterial, or agrochemical incorporating a microcapsule or a composition as hereinbefore described.

### DEFINITIONS

As used herein, the term "water soluble" refers to a substance having a solubility in water, measured at ambient temperature (e.g. 25 °C ± 2 °C) and at ambient pressure (e.g. 1013 mbar), at least equal to 1 gram/litre (g/L).

As used herein, the term "hydrophilic phase" refers to a phase in which a water-soluble ingredient is capable of dissolving.

As used herein, the term "oil soluble" refers to a substance having a solubility in in oil or organic solvent, measured at ambient temperature (e.g. 25 °C ± 2 °C) and at ambient pressure (e.g. 1013 mbar) at least equal to 1 gram/litre (g/L).

As used herein, the term "lipophilic phase" refers to a phase in which an oil-soluble ingredient is capable of dissolving.

As used herein, the term "primary emulsion" refers to a system wherein a hydrophilic phase is dispersed in an immiscible lipophilic phase.

As used herein, the term "double emulsion" refers to a primary emulsion, which has itself been dispersed in a further immiscible phase, e.g. a plant-based protein solution.

As used herein, the term "triple emulsion" refers to a double emulsion, which has itself been dispersed in a further immiscible phase, e.g. a lipophilic phase.

As used herein, the term "oil-soluble surfactant" refers to a compound that can lower the surface tension of a lipophilic phase.

### DETAILED DESCRIPTION

The present invention provides a microcapsule comprising:
(a) a hydrophilic phase comprising a water-soluble ingredient;
(b) a lipophilic phase; and
(c) a plant-based protein hydrogel shell.

The microcapsules of the present invention can be used to encapsulate a variety of substances and find various industrial applications, including in cosmetics, food use, household product use, agrochemical use and pharmaceutical use.

In preferred microcapsules of the present invention, the hydrophilic phase comprises water.

The microcapsules of the present invention comprise a water-soluble ingredient. Suitable water-soluble ingredients for use in the microcapsules of the present invention are described below. The concentration of the water-soluble ingredient in the hydrophilic phase will vary depending upon the solubility of the specific water-soluble ingredient being employed.

Suitable water-soluble ingredients include Vitamin B1, Vitamin B2, Vitamin B3, Vitamin B5, Vitamin B6, Vitamin B7, Vitamin B9, Vitamin B12, Vitamin C, panthenol, α-hydroxy acids, water-soluble minerals salts, water-soluble plant extracts and yeasts, enzymes, antibiotics, oligopeptides, proteins and protein hydrolysates.

In preferred microcapsules of the present invention, the lipophilic phase comprises an oil. Preferably, the oil is selected from plant-based oils (e.g. vegetable oils) and synthetic oils.

In preferred microcapsules of the present invention, the lipophilic phase further comprises an oil-soluble surfactant. Preferably, the oil-soluble surfactant is selected from sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.

Without wishing to be bound by theory, it is thought that the presence of an oil-soluble surfactant in the microcapsules of the present invention allows for the formation of a more stable primary emulsion because the oil-soluble surfactant can lower the surface tension of the lipophilic phase.

In preferred microcapsules of the present invention, the concentration of the oil-soluble surfactant in the lipophilic phase is in the range 0.01% w/w to 10% w/w, preferably in the range 0.1% w/w to 5% w/w, more preferably in the range 0.5% w/w to 2% w/w.

In preferred microcapsules of the present invention, the lipophilic phase further comprises an oil-soluble ingredient. Suitable oil-soluble ingredients for use in the microcapsules of the present invention are described below.

Further oil soluble ingredients include fatty acids; triglycerides or mixtures thereof; omega-3 fatty acids, such as a-linolenic acid (18: 3n3), octadecatetraenoic acid (18: 4n3), eicosapentaenoic acid (20: 5n3) (EPA) and docosahexaenoic acid (22: 6n3) (DHA), and derivatives thereof and mixtures thereof; fat-soluble vitamins, such as Vitamin A, Vitamin D, Vitamin E and Vitamin K; Antioxidants, such as tocopheryl and ascorbyl derivatives; retinoids or retinols; essential oils; bioflavonoids, terpenoids; synthetics of bioflavonoids and terpenoids and the like.

In preferred microcapsules of the present invention, the plant-based protein hydrogel shell is a self-assembled plant-based protein hydrogel shell.

The plant-based protein hydrogel employed as a shell in the microcapsules of the present invention is preferably made by a method comprising:
a) forming a solution comprising one or more plant-based protein(s) in a solvent system, wherein the solvent system comprises miscible co-solvents; wherein a first co-solvent increases solubility of the plant-based protein(s), and a second co-solvent decreases solubility of the plant-based protein(s); and
b) inducing the protein in the solution to undergo a sol-gel transition to form a plant-based protein hydrogel.

Any suitable plant-based proteins may be used. Suitable plant sources include soybean, pea, rice, potato, wheat, corn zein, sorghum, and the like. Particularly preferable plant proteins include soy proteins, pea proteins, potato proteins, rapeseed proteins and/or rice proteins, more preferably soy proteins and/or pea proteins.

Suitable plant-based proteins further include:
- Brassicas: including Brassica balearica: Mallorca cabbage, Brassica carinata: Abyssinian mustard or Abyssinian cabbage, Brassica elongata: elongated mustard, Brassica fruticulosa: Mediterranean cabbage, Brassica hilarionis: St Hilarion cabbage, Brassica juncea: Indian mustard, brown and leaf mustards, Sarepta mustard, Brassica napus: rapeseed, canola, rutabaga, Brassica narinosa: broadbeaked mustard, Brassica nigra: black mustard, Brassica oleracea: kale, cabbage, collard greens, broccoli, cauliflower, kai-lan, Brussels sprouts, kohlrabi, Brassica perviridis: tender green, mustard spinach, Brassica rapa (syn. B. campestris): Chinese cabbage, turnip, rapini, komatsuna, Brassica rupestris: brown mustard, Brassica tournefortii: Asian mustard
- Solanaceae: including tomatoes, potatoes, eggplant, bell and chili peppers;
- cereals: including maize, rice, wheat, barley, sorghum, millet, oats, rye, triticale, fonio
- pseudocereals: including amaranth (love-lies-bleeding, red amaranth, prince-of-Wales-feather), breadnut, buckwheat, chia, cockscomb (also called quail grass or soko), pitseed Goosefoot, qañiwa, quinoa and, wattleseed (also called acacia seed);
- Legume: including Acacia alata (Winged Wattle), Acacia decipiens, Acacia saligna (commonly known by various names including coojong, golden wreath wattle, orange wattle, blue-leafed wattle), Arachis hypogaea (peanut), Astragalus galegiformis, Cytisus laburnum (the common laburnum, golden chain or golden rain), Cytisus supinus, Dolichios lablab (common names include hyacinth bean, lablab-bean bonavist bean/pea, dolichos bean, seim bean, lablab bean, Egyptian kidney bean, Indian bean, bataw and Australian pea.), Ervum lens (Lentil), Genista tinctorial (common names include dyer's whin, waxen woad and waxen wood), Glycine max (Soybean), Lathyrus clymenum (peavines or vetchlings), Lathyrus odoratus (peavines or vetchlings), Lathyrus staivus (peavines or vetchlings), Lathyrus Silvetris (peavines or vetchlings), Lotus tetragonolobus (asparagus-pea or winged pea), Lupinus albus (Lupin), Lupinus angustifolius (lupin), Lupinus luteus (Lupin), Lupinus polyphyllus (Lupin), Medicago sativa (Alfalfa), Phaseolus aureus (Mung bean), Phaseolus coccineus (Runner bean), Phaseolus nanus (Green bean / French bean), Phaseolus vulgaris (Green bean / French bean), Pisum sativum (pea), Trifolium hybridum (Clover), Trifolium pretense (Red clover), Vicia faba (Broad bean), Vicia sativa (Vetch), Vigna unguiculate (cowpea)
- Non-Legumes: including: Acanshosicyos horrida (Acanshosicyos horrida), Aesculus hyppocastanum (Conker tree / Horsechestnut), Anacardium occidentale (Cashew tree), Balanites aegyptica, Bertholletia excels (Brazil nut), Beta vulgaris (Sugar beet), Brassica napus (Rapeseed), Brassica juncea (Brown mustard), Brassica nigra (Black mustard), Brassica hirta (Eurasian mustard), Cannabis sativa (marijuana), Citrullus vulgaris (Sort of watermelon), Citrus aurantiaca (Citrus), Cucurbita maxima (squash), Fagopyrum esculentum (knotweed), Gossypium barbadense (Extra long staple cotton), Heianthus annuus (sunflower), Nicotiana sp. (Tobacco plant), Prunus avium (cherry), Prunus cerasus (Sour cherry), Prunus domestica (plum), Prunus amygdalus (almond), Rricinus communis (Caster bean/ caster oil plant), Sasamum indicum (Sesame), Sinapis alba (White mustard), Terlfalrea pedata (Oyster nut).

For the avoidance of doubt, the plant-based microcapsules of the present invention do not encompass plants in their natural state, e.g. naturally formed plant cells, organelles or vesicles are not plant-based microcapsules of the present invention.

In the method described above, the plant-based protein hydrogel is formed by adding the plant-based protein into a solvent system, wherein the solvent system comprises two or more miscible co-solvents as defined herein.

The first co-solvent increases solubility of the plant-based protein(s). The first co-solvent may be considered a solubilising co-solvent. There may be one or more solubilising co-solvent(s) and the solubilising co-solvent(s) may fully or partially solubilise the plant-based protein(s).

Examples of solubilising co-solvents are organic acids. An organic acid is an organic compound with acidic properties. Suitable organic acids include acetic acid or an α-hydroxy acid. Suitable α-hydroxy acids include glycolic acid, lactic acid, malic acid, citric acid and tartaric acid. Preferred organic acids are acetic acid and lactic acid. Using an organic acid enables solubilisation of the plant protein and also allows for mild hydrolysis of the protein. For example, without wishing to be bound by theory, the solubility of plant-based proteins in organic acid is possible due to: i) the protonation of proteins and ii) the presence of an anion solvation layer which contributes to a reduction of hydrophobic interactions. Once initially dissolved in organic acid, the protonation of plant-based proteins can help to stabilise them in its non-solvent, for example water.

Preferably, the first co-solvent is an organic acid.

The second co-solvent has decreased solubility of the plant-based protein(s), as compared to the first co-solvent. The second co-solvent may be considered a de-solubilising co-solvent. There may be one or more de-solubilising co-solvent(s).

Examples of de-solubilising second co-solvent(s) are an aqueous buffer solution. In a further embodiment, the second co-solvent may be water, ethanol, methanol, acetone, acetonitrile, dimethylsulfoxide, dimethylformamide, formamide, 2-propanol, 1-butanol, 1- propanol, hexanol, t-butanol, ethyl acetate or hexafluoroisopropanol. In a particularly preferred embodiment, the second co-solvent is water and ethanol. In a further particularly preferred embodiment, the second co-solvent is water.

Preferably, the solvent system comprises a co-solvent ratio of about 20-80% v/v, preferably about 20-60% v/v, about 25-55% v/v, about 30-50% v/v, about 20%, about 30%, about 40% about 50% or about 60% v/v, most preferably about 30-50% v/v.

Preferably, the concentration of plant-based protein(s) in the solvent system is 25-200mg/ml, more preferably 50-150mg/ml. The ratio of organic acid may vary depending on protein concentration, e.g. using a higher organic acid ratio with increasing protein concentration.

Preferably, the degree of protein hydrolysis is controlled to modify the properties of the resultant hydrogel. For example, increasing the acid concentration present during formation will increase the degree of protein hydrolysis. Higher degree of protein hydrolysis leads to the formation of less rigid hydrogels.

In order to form the solution comprising one or more plant-based protein(s), it may be necessary to apply physical stimulus to the protein / solvent system mixture to enable dissolution of the protein. Suitable physical stimulus includes heating, ultrasonication, agitation, high-shear mixing or other physical techniques. A preferred technique is heating, optionally with subsequent ultrasonication.

Preferably, the protein / solvent system mixture is subjected to a physical stimulus which is heating, wherein the solution is heated to about or above 70°C. More preferably, the protein / solvent system mixture is heated to about or above 75°C, about or above 80°C, about or above 85°C or about 90°C. Even more preferably, the protein / solvent system mixture is heated to 85°C.

Preferably, the protein / solvent system mixture is subjected to a physical stimulus which is heating for a period of about 5 minutes, 10 minutes, 15 minutes, 20 minutes, 25 minutes, 30 minutes, or greater than 30 minutes. A preferred heating time period is about 30 minutes. The heated protein / solvent system mixture is optionally subjected to subsequent ultrasonication (e.g. for a period of up to 10 minutes).

The protein solution is then heated such that the liquid solution is held above the sol-gel transition for the protein(s). By modifying the solvent system (for example through selection of the choice of organic acid, the ratio of organic acid to further solvent or through further means) it is possible to modify the sol-gel transition temperature for the protein(s). Through appropriate selection of conditions, it is possible to carefully control the sol-gel transition of the protein thereby controlling the formation of the hydrogel.

Preferably, the protein solution is heated to about or above 70°C. More preferably, the protein is heated to about or above 75°C, about or above 80°C, about or above 85°C or about 90°C. Even more preferably, the protein is heated to 85°C.

The protein solution may be held at elevated temperature for a time period of about 5 minutes, 10 minutes, 15 minutes, 20 minutes, 25 minutes, 30 minutes, 45 minutes or 1 hour. A preferred time period is at least 30 minutes to enable the proteins to fully solubilise. It is possible to hold the protein solution at an elevated temperature for a longer period of time. This may be useful for a commercial batch process or for use in a fluidic processing step where it is necessary to retain the protein solution in liquid form for higher periods of time.

Having heated the protein solution to above the sol-gel transition temperature of the protein solution, the temperature of the protein solution can be reduced to a second temperature below the sol-gel transition temperature to facilitate formation of the hydrogel. The second temperature may be room temperature. The protein solution may be held at the reduced temperature for a time period of about 5 minutes, 10 minutes, 15 minutes, 20 minutes, 25 minutes or about 30 minutes. A particular reduced time period is about 5 minutes. However the method described above allows the protein to remain in solution for long periods of time. As such, depending on need, the protein solution can be kept above the sol-gel transition temperature for as long as required to retain the protein in liquid form. This could be hours, days or more. Also, since the reaction is reversible, a solution could, for example be kept at a lower temperature (for example room temperature) where a hydrogel will form but thereafter heated to above the sol-gel transition temperature to return the solution to a liquid state for further processing. Protein hydrogels in this way could be stored for hours, days, weeks, months or years as the hydrogel remains stable for a long time.

The particular temperatures will depend on the properties of the protein source, the solvent conditions used and therefore the sol-gel transition temperature. Alternatively, the elevated and reduced temperatures may be relatively fixed (for example about 85°C then about room temperature) and the co-solvent mixture conditions are adjusted to ensure a suitable sol-gel transition temperature for the selected plant-based protein.

Without wishing to be bound by theory, it is believed that when the plant protein is added to the solvent system the plant protein form a highly viscous dispersion of insoluble colloidal protein aggregates.

Further, it is believed that the application of mechanical agitation, for example ultrasonication, disrupts large colloidal protein aggregates into smaller ones, as well as disrupting protein intermolecular interactions. Using this approach, the size of the protein aggregates can be significantly reduced, before gelation, to particle sizes below 100nm. Preferably, the method described above comprises protein aggregates with an average size less than 200nm, preferably less than 150nm, less than 125nm, less than 100nm, less than 90nm, less than 80nm, less than 70nm, less than 60nm, less than 50nm, less than 40nm, or less than 30nm.

Aggregate size may be measured by Dynamic Light Scattering (DLS). Suitable apparatus to measure aggregate size is a Zetasizer Nano S (Malvern).

Further, it is believed that upon heating the protein solution in the presence of a co-solvent system to above the sol-gel temperature, the plant proteins partially unfold, resulting in the exposure of hydrophobic amino acids initially buried within the protein native structure. Once partially unfolded, the co-solvents are able to interact with the unfolded protein molecules. For example, an organic acid has greater access to protonate amino acid residues, as well as enabling the formation of anion salt bridges that stabilise hydrophobic interactions. Also, upon heating at elevated temperatures, protein-protein non-covalent intermolecular contacts are disrupted. Further, it is believed that upon cooling the protein solution to below the sol-gel temperature, protein-protein non-covalent intermolecular contacts are enabled, thus promoting the self-assembly of plant protein molecules into a network of supramolecular aggregates.

After gelation, the aggregates may be fine stranded. The aggregates may have a median average length of between 50 to 500nm. The aggregates may have a mean average length of between 50 to 500nm. 80% of the aggregates may have an average length of between 50 to 500nm. The aggregates may have a median height of between 5 to 50nm. The aggregates may have a mean average height of between 5 to 50nm. 80% of the aggregates may have an average height of between 5 to 50nm. In a preferred embodiment, the aggregates have a median average length of between 50 to 500nm and/or a median average height of between 5 to 50nm.

Without wishing to be bound by theory, it is thought that the method described above allows the plant proteins to aggregate into supramolecular structures held by intermolecular hydrogen bonding interactions, and in particular between the β-strands. Thus, preferably, the hydrogels employed as a shell in the microcapsules of the present invention has high levels of β-sheet intermolecular interactions.

Preferably, the hydrogels employed as a shell in the microcapsules of the present invention have a protein secondary structure with at least 40% intermolecular β-sheet, at least 50% intermolecular β-sheet, at least 60% intermolecular β-sheet, at least 70% intermolecular β-sheet, at least 80% intermolecular β-sheet, or at least 90% intermolecular β-sheet. It is believed that previous gels made from plant-based protein sources may have lower amounts of intermolecular β-sheets in the secondary structure, leading to prior art disadvantageous properties.

The plant-based protein hydrogel employed as a shell in the microcapsules of the present invention has advantageous mechanical properties. For example, the ability to reversibly change from gel to liquid upon temperature change enables advantageous manufacturing capabilities.

Preferably, the hydrogels employed as a shell in the microcapsules of the present invention have a storage modulus (G') at 10rad/s of greater than 500Pa, greater than 1000Pa, greater than 2500Pa, greater than 3000Pa, greater than 4000Pa.

Preferably, the hydrogels employed as a shell in the microcapsules of the present invention exhibit shear-thinning behaviour, where the viscosity decreases upon increasing shear rates.

Preferably, the hydrogels employed as a shell in the microcapsules of the present invention are not adversely affected by processing conditions normally present during final product manufacturing processes, e.g. exposure to high temperatures (e.g. > 95°C) and/or high shear conditions. More preferably, the hydrogels employed as a shell in the microcapsules of the present invention are chemically and/or mechanically stable to such processing conditions.

Preferably, the hydrogels employed as a shell in the microcapsules of the present invention are stable under low pH conditions. More preferably, the hydrogels employed as a shell in the microcapsules of the present invention are stable at a pH of less than 3 (e.g. at a pH of less than 2). This is particularly advantageous when the microcapsules are to be employed in a food, beverage or pharmaceutical product. Preferably, the hydrogels employed as a shell in the microcapsules of the present invention exhibit a unique thermoreversible gelling behaviour. Upon heating at elevated temperatures and/or by applying mechanical agitation, the protein gels return to liquid form. This is a unique property that has not been seen with prior hydrogels, which would not upon heating return to a fully liquid state. In contrast, hydrogels employed as a shell in the microcapsules of the present invention can. Preferably, upon heating at elevated temperatures and/or by applying mechanical agitation, protein solutions may have a storage modulus lower than 250Pa, lower than 100Pa, lower than 50Pa, lower than 10Pa. This enables the microcapsules of the present invention to have unique manufacturing capabilities.

Equally, if desired, thermoreversibility can be removed by removing the solvent system. Once the plant-based microcapsules of the present invention have been formed, the solvent system can be washed out which will stop the thermoreversible properties of the microcapsules. Thus, if the microcapsule is thereafter heated it will remain stable and will not re-melt. This enables, for example, microcapsules according to the present invention to be subjected to high temperature processes yet remain intact and stable.

Thus, the hydrogels employed as a shell in the microcapsules of the present invention have unique properties not seen in prior plant-based hydrogels. These include the ability of forming hydrogels from plant-based proteins at high concentrations (i.e. 5%-15% w/w) from commercially available sources, and the ability of keeping such highly concentrated protein solutions in the liquid state upon heat denaturation, which enables their moulding into well-defined objects.

A feature of the hydrogels employed as a shell in the microcapsules of the present invention is that it is not necessary to provide cross-linking agents as the plant proteins will self-form hydrogels. Thus, preferably the hydrogels employed as a shell in the microcapsules of the present invention do not contain or do not substantially contain a cross-linking agent.

However, in an alternative embodiment, the hydrogels employed as a shell in the microcapsules of the present invention may comprise a cross-linking agent. Suitable cross-linking agents include microbial transglutaminase, glutaraldehyde, formaldehyde, glyoxal, phenolic compounds, epoxy compounds, genipin or dialdehyde starch.

Due to the porous network of the hydrogel, the solvent mixture within the hydrogel can be exchanged for another solvent mixture without compromising the mechanical stability of the hydrogel. A solvent-exchange process can be performed to remove the organic acid from the hydrogel porous network.

Preferably, the hydrogels employed as a shell in the microcapsules of the present invention can incorporate one or more plasticizers. Possible plasticizers include ethylene glycol, diethylene glycol, triethylene glycol, tetraethylene glycol, polyethylene glycol, propylene glycol, sorbitol, mannitol, xylitol, fatty acids, glucose, mannose, fructose, sucrose, ethanolamine, urea, triethanolamine; vegetable oils, lecithin, waxes and amino acids.

Preferably the hydrogel may comprise about 1% plasticiser, about 2%, about 5%, about 10%, about 20%, about 30%, about 40%, about 50%, about 60% or more. Alternatively, the hydrogel may comprise between about 5-50% plasticiser, about 10-50%, about 20-40% about 15-35% or about 20% plasticizer.

Adding a plasticizer can influence the mechanical properties of the material. Typically, adding a plasticiser will increase the elasticity of the material but this typically conversely reduces the strength of the resultant material.

The use of plant-based hydrogels as a shell in the microcapsules of the present invention has a number of advantages over previously used animal or petrochemical sources. Firstly, plant sources are renewable and can be efficiently obtained in an environmentally efficient manner. Secondly, plant sources are biodegradable and are therefore an environmentally sound alternative to other plastics. Thirdly, in contrast to animal derived proteins, plant-based proteins have the significant advantage that they do not introduce animal derived proteins into a human. This has positive impacts both from a pharmacological and pharmaceutical perspective where animal sourced material must undergo stringent checks and processes to ensure no adverse elements are present (for example removing prions and the like); but also because the products are suitable for vegetarian/vegans.

Since plant-based proteins are naturally present in a human (or other animal)'s diet, the plant-based hydrogels employed as a shell in the microcapsules of the present invention exhibit a higher degree of digestibility compared to other biopolymers such as polysaccharides (for example, alginates or chitosan). This makes them particularly suitable for pharmaceutical, food and/or cosmetic use.

In preferred microcapsules of the present invention, the hydrophilic phase is dispersed in the lipophilic phase.

In preferred microcapsules of the present invention, the hydrophilic phase and the lipophilic phase are encapsulated by the plant-based protein hydrogel shell.

In preferred microcapsules of the present invention, the hydrophilic phase and the lipophilic phase form a water-in-oil emulsion.

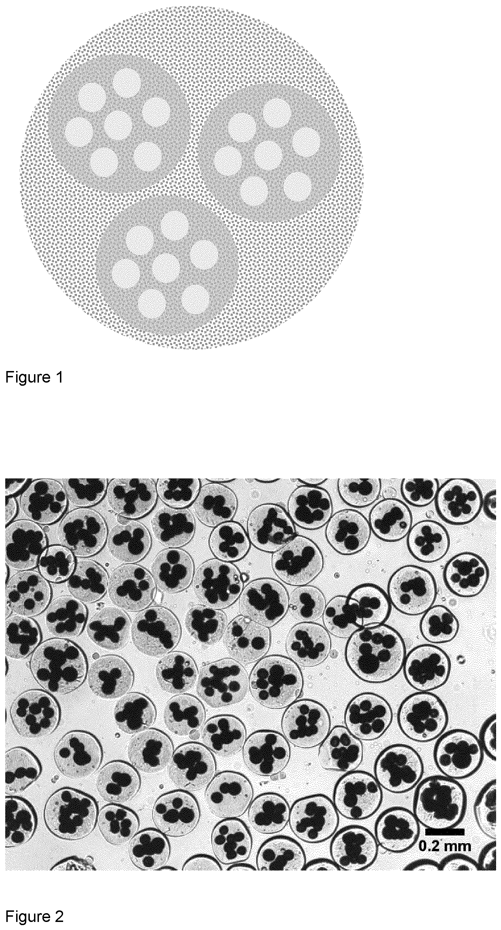

In preferred microcapsules of the present invention, the water-in-oil emulsion is encapsulated by the plant-based protein hydrogel shell. Such an embodiment is depicted graphically in Figure 1.

The microcapsules of the present invention encapsulate water-soluble ingredients. The microcapsules of the present invention optionally also encapsulate oil-soluble ingredients.

In preferred microcapsules of the present invention, the plant-based protein hydrogel shell can encapsulate any nutraceutical, cosmetic, pharmaceutical or agrochemical suitable active agent including vitamins, essential fatty acids, antioxidants, small molecules, hydrophilic small molecules, hydrophobic small molecules, proteins, antibodies, antibody-drug conjugates, fragrances and other large molecules.

Suitable encapsulated agents include one or more agents selected from:
cross-linkers, hardeners, organic catalysts and metal-based catalysts (for example organo-complexes and inorgano-complexes of platinum, palladium, titanium, molybdenum, copper, or zinc) for polymerization of elastomer formulations, rubber formulations, paint formulations, coating formulations, adhesive formulations, or sealant formulations;
dyes, colorants, pigments for inks, personal care products, elastomer formulations, rubber formulations, paint formulations, coating formulations, adhesive formulations, sealant formulations, or paper formulations;
fragrances for detergents, housecleaning products, personal care products, textiles (so-called smart textiles), coating formulations. Fragrances useful to the invention are any of the compounds belonging to the list of standards published and updated by the International Fragrance Association (IFRA);
aromas, flavors, vitamins, aminoacids, proteins, essential lipids, probiotics, antioxidants, preservatives for feed and food products;
fabric softeners and conditioners for detergents and personal care products;.
bioactive compounds such as enzymes, vitamins, proteins, vegetable extracts, moisturizers, sanitizers, antibacterial agents, sunscreen agents, drugs, for personal care products, textiles (so-called smart textiles). These compounds include but are not limited to vitamin A, vitamin B, vitamin C, vitamin D, vitamin E, para aminobenzoic acid, alpha hydroxyacid, camphor, ceramides, ellagic acid, glycerin, glycin, glycolic acid, hyaluronic acid, hydroquinone, isopropyl, isostearate, isopropyl palmitate, oxybenzone, panthenol, proline, retinol, retinyl palmitate, salicylic acid, sorbic acid, sorbitol, triclosan, tyrosine; and
fertilizers, herbicides, insecticides, pesticides, fungicides, repellants, and disinfectants for agrochemicals. A person skilled in the art would understand which of the above listed encapsulated agents would be water-soluble ingredients and which would be oil-soluble ingredients for the purposes of the present invention.

In preferred microcapsules of the present invention, the plant-based protein hydrogel shell has a thickness in the range 10 nm to 50,000 µm, preferably in the range 10 µm to 100 µm, more preferably in the range 10µm to 50µm, most preferably in the range 1µm to 10µm.

In preferred microcapsules of the present invention, the plant-based protein hydrogel shell has a thickness of about 100nm, 200nm, 300nm, 400nm, 500nm, 600nm, 700nm, 800nm, 900nm, 1µm , 2µm, 3µm, 4µm, 5µm, 6µm, 7µm, 8µm, 9µm, 10 µm, 11 µm, 12 µm, 13 µm, 14 µm, 15 µm, 16 µm, 17 µm, 18 µm, 19 µm, 20 µm, 25 µm, 30 µm, 30 µm, 35 µm, 40 µm, or 50 µm.

In preferred microcapsules of the present invention, the microcapsule has a size of less than 1mm in the largest dimension, less than 900 µm, less than 800 µm, less than 700 µm, less than 600 µm, less than 500 µm, less than 400 µm, less than 300 µm, less than 200 µm, or less than 100 µm.

In preferred microcapsules of the present invention, the microcapsule releases the water-soluble ingredient and/or the oil-soluble ingredient to a surface upon application of pressure to the microcapsule (e.g. the application of pressure breaks the plant-based protein hydrogel shell). Preferably, the surface is a bio-surface. More preferably, the bio-surface is selected from hair, skin and teeth. Alternatively, the surface is a textile. The porous nature of the plant-based protein hydrogel shell allows for a controlled release of the water-soluble ingredient and/or the oil-soluble ingredient from the microcapsule.

In preferred microcapsules of the present invention, the microcapsule releases the water-soluble ingredient and/or the oil-soluble ingredient as a result of enzymatic degradation of the plant-based protein hydrogel shell. Preferably, said enzymatic degradation occurs in the digestive system of a human or animal.

The present invention also provides a composition comprising at least one microcapsule as hereinbefore described and an external phase.

In preferred compositions of the present invention, the at least one microcapsule is dispersed in the external phase.

In preferred compositions of the present invention, the external phase is an external aqueous phase (e.g. an aqueous salt solution). Preferably, the external aqueous phase is a continuous external aqueous phase. In alternative preferred compositions of the present invention, the external phase is an external lipophilic phase.

Without wishing to be bound by theory, it is thought that control of the relative ionic potential inside and outside the microcapsules can allow for adjustment of the osmotic pressure across the microcapsules. In turn, this can allow for control over the release properties of ingredients from the microcapsules (e.g. controlled or delayed release of the water-soluble ingredient(s) from the microcapsules).

The present invention also provides method for preparing a microcapsule as hereinbefore described, comprising:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein, to give a double emulsion;
(c) re-emulsifying said double emulsion in a second lipophilic phase to give a triple emulsion;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase; and
(e) washing the microcapsule to remove the second lipophilic phase.

In preferred methods of the invention, said plant-based protein solution comprises one or more plant-based protein(s) in a solvent system, wherein the solvent system comprises miscible co-solvents; wherein a first co-solvent increases solubility of the plant-based protein(s), and a second co-solvent decreases solubility of the plant-based protein(s). Preferred features of the plant-based protein(s), the first co-solvent and the second co-solvent are discussed above.

In preferred methods of the present invention, the primary emulsion has a diameter that is less than or equal to 5 µm, preferably less than or equal to 4 µm, more preferably less than or equal to 3 µm, even more preferably less than or equal to 2 µm, most preferably less than or equal to 1 µm.

In preferred methods of the present invention, the double emulsion has a diameter that is less than or equal to 100 µm, preferably less than or equal to 50µm, more preferably less than or equal to 30 µm.

In preferred methods of the present invention, the triple emulsion has a diameter that is less than or equal to 200 µm, preferably less than or equal to 150 µm, more preferably less than or equal to 100 µm.

In preferred methods of the present invention, the first and second lipophilic phases are the same (e.g. they are the same type of oil).

In alternative preferred methods of the present invention, the first and second lipophilic phases are different (e.g. they are different types of oils).

In preferred methods of the present invention, the first and/or second lipophilic phase further comprises an oil-soluble surfactant. Preferably, the oil-soluble surfactant(s) is selected from sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.

In preferred methods of the present invention, the oil-soluble surfactants in the first and second lipophilic phase are the same.

In alternative preferred methods of the present invention, the oil-soluble surfactants in the first and second lipophilic phase are different.

In preferred methods of the present invention, said microcapsule is formed using a microfluidic device.

Preferred methods of the present invention further comprise drying the microcapsule to form a dry powder. Preferably, said drying is selected from spray drying, fluid bed drying and/or tray drying. The drying step allows for ease of handling of the microcapsules, for example by avoiding the need to transport the final microcapsules in large quantities of water.

The present invention also provides a microcapsule prepared according to the method hereinbefore described.

An alternative method for preparing a microcapsule as hereinbefore described comprises:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein solution, to give a double emulsion;
(c) subjecting the plant-based protein solution of said double emulsion to an aqueous phase separation step or a coacervation step;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase; and
(e) washing the microcapsule to remove the external phase.

The present invention also provides a method for preparing a composition as hereinbefore described, comprising:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein, to give a double emulsion;
(c) re-emulsifying said double emulsion in a second lipophilic phase to give a triple emulsion;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase.

Preferred methods of the present invention further comprise:
(e) washing the microcapsule to remove the second lipophilic phase; and
(f) re-suspending the microcapsule in an external aqueous phase.

In preferred methods of the invention, said plant-based protein solution comprises one or more plant-based protein(s) in a solvent system, wherein the solvent system comprises miscible co-solvents; wherein a first co-solvent increases solubility of the plant-based protein(s), and a second co-solvent decreases solubility of the plant-based protein(s). Preferred features of the plant-based protein(s), the first co-solvent and the second co-solvent are discussed above.

In preferred methods of the present invention, the primary emulsion has a diameter that is less than or equal to 5 µm, preferably less than or equal to 4 µm, more preferably less than or equal to 3 µm, even more preferably less than or equal to 2 µm, most preferably less than or equal to 1 µm.

In preferred methods of the present invention, the double emulsion has a diameter that is less than or equal to 100 µm, preferably less than or equal to 50 µm, more preferably less than or equal to 30 µm.

In preferred methods of the present invention, the triple emulsion has a diameter that is less than or equal to 200 µm, preferably less than or equal to 150 µm, more preferably less than or equal to 100 µm.

In preferred methods of the present invention, the second lipophilic phase comprises an oil. Preferably, the oil is selected from plant-based oils (e.g. vegetable oils) and synthetic oils.

In preferred methods of the present invention, the first and second lipophilic phases are the same (e.g. they are the same type of oil).

In alternative preferred methods of the present invention, the first and second lipophilic phases are different (e.g. they are different types of oils).

In preferred methods of the present invention, the first and/or second lipophilic phase further comprises an oil-soluble surfactant. Preferably, the oil-soluble surfactant(s) is selected from sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.

In preferred methods of the present invention, the oil-soluble surfactants in the first and second lipophilic phase are the same.

In alternative preferred methods of the present invention, the oil-soluble surfactants in the first and second lipophilic phase are different.

Preferred methods of the present invention further comprise adding suspending agents to said external aqueous phase. Preferably, said suspending agents are selected from acacia gum, alginic acid, pectin, xanthan gum, gellan gum, carbomer, dextrin, gelatin, guar gum, hydrogenated vegetable oil category 1, aluminum magnesium silicate, maltodextrin, carboxymethyl cellulose, polymethacrylate, poly vinyl pyrrolidone, sodium alginate, starch, zein, water-insoluble cross-linked polymers such as cross-linked cellulose, cross-linked starch, cross-linked CMC, cross-linked carboxymethyl starch, cross-linked polyacrylate, and cross-linked polyvinylpyrrolidone, and expanded clays such as bentonite and laponite.

In preferred methods of the present invention, said microcapsule is formed using a microfluidic device.

The present invention also provides a composition prepared according to the method hereinbefore described.

An alternative method for preparing a composition as hereinbefore described comprises:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein solution, to give a double emulsion;
(c) subjecting the plant-based protein solution of said double emulsion to an aqueous phase separation step or a coacervation step;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase.

Preferably, the alternative method further comprises:
(e) washing the microcapsule to remove the external phase; and
(f) re-suspending the microcapsule in an external aqueous phase.

The present invention also provides a food, beverage, cosmetic, pharmaceutical, medical device, biomaterial, or agrochemical incorporating a microcapsule as hereinbefore described, or a composition as hereinbefore described.

### BRIEF DESCRIPTION OF THE FIGURES

Figure 1 is a graphical representation of an embodiment of the microcapsules of the present invention.
Figure 2 is a photograph of a composition comprising the microcapsules of Example 1 of the present invention.

### EXAMPLES

### Materials

- Pea Protein Isolate (PPI) (80% protein) and lactic acid (80%) were purchased from Cambridge Commodities Ltd.
- Calcium chloride dihydrate was purchased from Fisher Scientific.
- Miglyol 840 and polyglycerol polyricinoleate (PGPR) were purchased from IOI Oleo.
- (1H,1H,2H,2H-Perfluoro-1-octanol) was purchased from Sigma Aldrich.
- HFE-7500 3M^{™} Novec^{™} Engineered fluid was purchased from Fluorochem.
- 008-FluoroSurfactant was purchased from RAN Biotechnologies.

### Example 1 - Preparation of microcapsules

### • Preparation of internal phase:

A water-in-oil emulsion was produced by emulsifying 200µl of a 0.25M CaCl₂ solution in 800µl of Miglyol 840 containing 4% (w/w) PGPR by ultrasonication for 30 seconds.

### • Preparation of middle phase:

Pea Protein Isolate was added to a 35%(v/v) lactic acid aqueous solution at a final protein concentration of 125 mg/ml. A dispersion of non-soluble protein was obtained. For solubilisation of the protein, the mixture was exposed to ultrasonication for 30 min (using a Bandelin Sonopuls HD 4200 Ultrasonic Homogeniser operating under the following parameters: High-Frequency Power Output=200W, Frequency=20KHz, Amplitude=20%). During this process, the sample temperature was kept at 85°C-90°C. After 30 min, a completely translucent liquid solution was obtained. The dispersed liquid PPI phase (125mg/ml PPI in 35% v/v acetic acid, kept at 85°C) was loaded in a 15ml tube and rapidly placed on a heating block at 85°C. In order to prevent the gelation of the PPI solution during transport into the microfluidic device, a custom-made silicone heater (Holroyd Components), comprising a 1/32" ID stainless steel tubing was used to maintain the temperature of the PTFE tubing connecting the PPI reservoir and the inlet in the microfluidic device. The silicon heater temperature was controlled by a custom-built temperature controller

### • Preparation of external oil phase:

008-FluoroSurfactant was dissolved in HFE-7500 3M^{™} Novec^{™} to a final concentration of 2% (w/w).

### • Preparation of microcapsules:

Non-planar microfluidic devices (droplet generators) were fabricated using standard soft lithography techniques with a negative master photoresist (SU8 3050), as described in Biomacromolecules 2017, 18, 11, 3642-3651. For the fabrication of a triple emulsion, a tandem emulsification approach was followed. The first non-planar droplet generator device was comprised of an internal channel (50x50µm), and an external channel (100x100 µm), and was rendered hydrophilic by exposure to oxygen plasma for 500 seconds and 80W. The second non-planar droplet generator microfluidic device was comprised of an internal channel (200x200µm) and an external channel (300x300µm), and was rendered hydrophobic by flushing all channels with a solution of Duxback^{®}. Both devices were connected via a small 1/32" OD PTFE tubing.

Droplets of -50 µm diameter were first generated by pumping the internal and middle phase into the hydrophilic device by a pressure-driven system (Elveflow OB1), thus obtaining a double emulsion. The generated droplets were transferred to the hydrophobic device and emulsified again by the outer phase, thus obtaining a triple emulsion.

Different pressure rates were tested until a uniform and continuous generation of a monodisperse population of microdroplets was achieved. Final pressure rates were 350 mbar for the internal phase, 500 mbar for the middle protein phase and 180mbar for the external oil phase. The generated droplets were collected in a glass vial, and were kept at 10°C for 12h to ensure the gelation process was complete.

The formed microcapsules were then washed by a standard de-emulsification procedure: the continuous oil phase containing fluorosurfactant was first removed from the vial. For 500ul of microcapsules, an equal volume of 10% PFO solution in HFE-7500 3M^{™} Novec^{™} was added and thoroughly mixed for 30 seconds. The 10% PFO solution in HFE-7500 3M^{™} Novec^{™} was then removed and two subsequent oil washes were performed by adding an equal volume of pure HFE-7500 3M^{™} Novec^{™}. Finally, 2500µl of a 0.1M CaCl₂ solution were added to the vial, resulting in the transfer of the microcapsules from the oil to the aqueous phase. The supernatant containing the microcapsules suspension was transferred to a separate vial. Figure 2 is a photograph of the microcapsule composition of Example 1.

### CLAUSES

1. A microcapsule comprising:
   (a) a hydrophilic phase comprising a water-soluble ingredient;
   (b) a lipophilic phase; and
   (c) a plant-based protein hydrogel shell.
2. A microcapsule according to clause 1, wherein the hydrophilic phase comprises water.
3. A microcapsule according to clause 1 or clause 2, wherein the water-soluble ingredient is selected from one or more of Vitamin B1, Vitamin B2, Vitamin B3, Vitamin B5, Vitamin B6, Vitamin B7, Vitamin B9, Vitamin B12, Vitamin C, panthenol, α-hydroxy acids, water-soluble minerals salts, water-soluble plant extracts and yeasts, enzymes, antibiotics, oligopeptides, proteins and protein hydrolysates.
4. A microcapsule according to any one of clauses 1 to 3, wherein the lipophilic phase comprises an oil.
5. A microcapsule according to any one of clauses 1 to 4, wherein the lipophilic phase further comprises an oil-soluble surfactant.
6. A microcapsule according to clause 5, wherein the oil-soluble surfactant is selected from sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.
7. A microcapsule according to clause 5 or clause 6, wherein the concentration of the oil-soluble surfactant in the lipophilic phase is in the range 0.01% w/w to 10% w/w.
8. A microcapsule according to any one of clauses 1 to 7, wherein the lipophilic phase further comprises an oil-soluble ingredient.
9. A microcapsule according to clause 8, wherein the oil-soluble ingredient is selected from one or more of: a fatty acid; a triglyceride or a mixture thereof; Omega-3 fatty acids, such as a-linolenic acid (18: 3n3), octadecatetraenoic acid (18: 4n3), eicosapentaenoic acid (20: 5n3) (EPA) and docosahexaenoic acid (22: 6n3) (DHA), and derivatives thereof and mixtures thereof; fat-soluble vitamins, such as Vitamin A, Vitamin D, Vitamin E and Vitamin K; Antioxidants, such as tocopheryl and ascorbyl derivatives; retinoids or retinols; essential oils; bioflavinoids, terpenoids; synthetics of bioflavonoids and terpenoids and the like.
10. A microcapsule according to any one of clauses 1 to 9, wherein the plant-based protein(s) in the plant-based protein hydrogel shell is obtained from soybean, pea, rice, potato, wheat, corn zein or sorghum; preferably the plant protein(s) is selected from soy protein, pea protein, potato protein, rapeseed protein and/or rice protein.
11. A microcapsule according to any one of clauses 1 to 10, wherein the plant-based protein hydrogel shell is a self-assembled plant-based protein hydrogel shell.
12. A microcapsule according to any one of clauses 1 to 11, wherein the plant-based protein hydrogel shell comprises plant-based proteins having a protein secondary structure with at least 40% intermolecular β-sheet, at least 50% intermolecular β-sheet, at least 60% intermolecular β-sheet, at least 70% intermolecular β-sheet, at least 80% intermolecular β-sheet, or at least 90% intermolecular β-sheet.
13. A microcapsule according to any one of clauses 1 to 12, wherein the plant-based protein hydrogel shell has a storage modulus (G') at 10rad/s of greater than 500Pa, greater than 1000Pa, greater than 2500Pa, greater than 3000Pa, greater than 4000Pa.
14. A microcapsule according to any one of clauses 1 to 13, wherein the plant-based protein hydrogel shell comprises protein aggregates with a median average length of between 50 to 500nm or a mean average length of between 50 to 500nm; or 80% of the aggregates have an average length of between 50 to 500nm;
   and/or the aggregates may have a median height of between 5 to 50nm; or the aggregates may have a mean average height of between 5 to 50nm; or 80% of the aggregates have an average height of between 5 to 50nm;
   preferably, the aggregates have a median average length of between 50 to 500nm and/or a median average height of between 5 to 50nm.
15. A microcapsule according to any one of clauses 1 to 14, wherein the hydrophilic phase is dispersed in the lipophilic phase.
16. A microcapsule according to any one of clauses 1 to 15, wherein the hydrophilic phase and the lipophilic phase form a water-in-oil emulsion.
17. A microcapsule according to any one of clauses 1 to 15, wherein the hydrophilic phase and the lipophilic phase are encapsulated by the plant-based protein hydrogel shell.
18. A microcapsule according to clause 16, wherein the water-in-oil emulsion is encapsulated by the plant-based protein hydrogel shell.
19. A microcapsule according to any one of clauses 1 to 18, wherein the plant-based protein hydrogel shell has a thickness in the range 10 nm to 50,000 µm, preferably in the range 10 µm to 100 µm.
20. A microcapsule according to any one of clauses 1 to 19, wherein the microcapsule has a size of less than 1mm in the largest dimension, preferably less than 900 µm.
21. A microcapsule according to any one of clauses 1 to 20, wherein the microcapsule releases the water-soluble ingredient and/or the oil-soluble ingredient to a surface upon application of pressure to the microcapsule.
22. A microcapsule according to clause 21, wherein said surface is a bio-surface.
23. A microcapsule according to clause 22, wherein said bio-surface is selected from hair, skin and teeth.
24. A microcapsule according to clause 21, wherein said surface is a textile.
25. A microcapsule according to any one of clauses 1 to 20, wherein the microcapsule releases the water-soluble ingredient and/or the oil-soluble ingredient as a result of enzymatic degradation of the plant-based protein hydrogel shell.
26. A microcapsule according to clause 25, wherein said enzymatic degradation occurs in the digestive system of a human or animal.
27. A composition comprising at least one microcapsule according to any one of clauses 1 to 26 and an external phase.
28. A composition according to clause 27, wherein the at least one microcapsule is dispersed in the external phase.
29. A composition according to clause 27 or clause 28, wherein the external phase is an external aqueous phase.
30. A composition according to clause 29, wherein the external aqueous phase is an aqueous salt solution.
31. A composition according to clause 29 or clause 30, wherein the external aqueous phase is a continuous external aqueous phase.
32. A composition according to clause 27 or clause 28, wherein the external phase is an external lipophilic phase.
33. A method for preparing a microcapsule according to any one of clauses 1 to 26, comprising:
   (a) emulsifying a hydrophilic phase comprising a water-soluble ingredient in a first lipophilic phase to give a primary emulsion;
   (b) re-emulsifying said primary emulsion in a plant-based protein solution comprising one or more plant-based protein(s), wherein said plant-based protein solution is at a temperature above the sol-gel transition temperature of the plant-based protein solution, to give a double emulsion;
   (c) re-emulsifying said double emulsion in a second lipophilic phase to give a triple emulsion;
   (d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase; and
   (e) washing the microcapsule to remove the second lipophilic phase.
34. A method according to clause 33, wherein the primary emulsion has a diameter of less than or equal to 5 µm.
35. A method according to clause 33 or clause 34, wherein the double emulsion has a diameter of less than or equal to 100 µm.
36. A method according to any one of clauses 33 to 35, wherein the triple emulsion has a diameter of less than or equal to 200 µm.
37. A method according to any one of clauses 33 to 36, wherein the first and second lipophilic phases are the same or different.
38. A method according to any one of clause 33 to 37, wherein the first and/or second lipophilic phase further comprises an oil-soluble surfactant.
39. A method according to clause 38, wherein the oil-soluble surfactants in the first and second lipophilic phase are the same or different.
40. A method according to any one of clauses 33 to 39, wherein the plant-based protein solution comprises one or more plant-based protein(s) in a solvent system, wherein the solvent system comprises miscible co-solvents; wherein a first co-solvent increases solubility of the plant-based protein(s), and a second co-solvent decreases solubility of the plant-based protein(s).
41. A method according to clause 40, wherein the first co-solvent is an organic acid; preferably acetic acid and/or an α-hydroxy acid; wherein the α-hydroxy acid may preferably be selected from glycolic acid, lactic acid, malic acid, citric acid and/or tartaric acid; with particularly preferred organic acids being acetic acid and/or lactic acid.
42. A method according to clause 40 or 41, wherein the second co-solvent(s) is an aqueous buffer solution, preferably selected from water, ethanol, methanol, acetone, acetonitrile, dimethylsulfoxide, dimethylformamide, formamide, 2-propanol, 1-butanol, 1-propanol, hexanol, t-butanol, ethyl acetate, hexafluoroisopropanol, more preferably water and/or ethanol, particularly preferably water.
43. A method according to any one of clauses 40 to 42, wherein said solvent system comprises a co-solvent ratio of about 20-80% v/v, preferably about 20-60% v/v, about 25-55% v/v, about 30-50% v/v, about 20%, about 30%, about 40% about 50% or about 60% v/v, most preferably about 30-50% v/v.
44. The method according to any one of clauses 33 to 43, wherein in step (d) the protein solution is heated to a first temperature above the sol-gel temperature of the one or more plant-based protein(s), then reduced to a second temperature below the sol-gel temperature of the one or more plant-based protein(s) to form the plant-based protein hydrogel shell.
45. The method according to any one of clauses 33 to 44 wherein said microcapsule is formed using a microfluidic device.
46. A method according to any one of clauses 33 to 45, further comprising drying the microcapsule to form a dry powder.
47. A method according to clause 46, wherein said drying is selected from spray drying, fluid bed drying and/or tray drying.
48. A microcapsule prepared according to the method of any one of clauses 33 to 47.
49. A method for preparing a composition according to any one of clauses 27 to 32, comprising:
   (a) emulsifying a hydrophilic phase comprising a water-soluble ingredient in a first lipophilic phase to give a primary emulsion;
   (b) re-emulsifying said primary emulsion in a plant-based protein solution comprising one or more plant-based protein(s), wherein said plant-based protein solution is at a temperature above the sol-gel transition temperature of the plant-based protein, to give a double emulsion;
   (c) re-emulsifying said double emulsion in a second lipophilic phase to give a triple emulsion;
   (d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase.
50. A method according to clause 49, wherein the primary emulsion has a diameter of less than or equal to 5 µm.
51. A method according to clause 49 or clause 50, wherein the double emulsion has a diameter of less than or equal to 100 µm.
52. A method according to any one of clauses 49 to 51, wherein the triple emulsion has a diameter of less than or equal to 200 µm.
53. A method according to any one of clauses 49 to 52, wherein the first and second lipophilic phases are the same or different.
54. A method according to any one of clauses 49 to 53, wherein the first and/or second lipophilic phase further comprises an oil-soluble surfactant.
55. A method according to clause 54, wherein the oil-soluble surfactants in the first and second lipophilic phase are the same or different.
56. A method according to any one of clauses 49 to 55, further comprising:
   (e) washing the microcapsule to remove the second lipophilic phase; and
   (f) re-suspending the microcapsule in an external aqueous phase.
57. A method according to clause 56, further comprising adding suspending agents to said external aqueous phase.
58. A method according to clause 57, wherein said suspending agents are selected from acacia gum, alginic acid, pectin, xanthan gum, gellan gum, carbomer, dextrin, gelatin, guar gum, hydrogenated vegetable oil category 1, aluminum magnesium silicate, maltodextrin, carboxymethyl cellulose, polymethacrylate, poly vinyl pyrrolidone, sodium alginate, starch, zein, water-insoluble cross-linked polymers such as cross-linked cellulose, cross-linked starch, cross-linked CMC, cross-linked carboxymethyl starch, cross-linked polyacrylate, and cross-linked polyvinylpyrrolidone, and expanded clays such as bentonite and laponite.
59. A method according to any one of clauses 49 to 58, wherein the plant-based protein solution comprises one or more plant-based protein(s) in a solvent system, wherein the solvent system comprises miscible co-solvents; wherein a first co-solvent increases solubility of the plant-based protein(s), and a second co-solvent decreases solubility of the plant-based protein(s).
60. A method according to clause 59, wherein the first co-solvent is an organic acid; preferably acetic acid and/or an α-hydroxy acid; wherein the α-hydroxy acid may preferably be selected from glycolic acid, lactic acid, malic acid, citric acid and/or tartaric acid; with particularly preferred organic acids being acetic acid and/or lactic acid.
61. A method according to clause 59 or clause 60, wherein the second co-solvent(s) is an aqueous buffer solution, preferably selected from water, ethanol, methanol, acetone, acetonitrile, dimethylsulfoxide, dimethylformamide, formamide, 2-propanol, 1-butanol, 1-propanol, hexanol, t-butanol, ethyl acetate, hexafluoroisopropanol, more preferably water and/or ethanol, particularly preferably water.
62. A method according to any one of clauses 59 to 61, wherein said solvent system comprises a co-solvent ratio of about 20-80% v/v, preferably about 20-60% v/v, about 25-55% v/v, about 30-50% v/v, about 20%, about 30%, about 40% about 50% or about 60% v/v, most preferably about 30-50% v/v.
63. The method according to any one of clauses 49 to 62, wherein in step (d) the protein solution is heated to a first temperature above the sol-gel temperature of the one or more plant-based protein(s), then reduced to a second temperature below the sol-gel temperature of the one or more plant-based protein(s) to form the plant-based protein hydrogel shell.
64. The method according to any one of clauses 49 to 63 wherein said microcapsule is formed using a microfluidic device.
65. A composition prepared according to the method of any one of clauses 49 to 64.
66. A food, beverage, cosmetic, pharmaceutical, medical device, biomaterial, or agrochemical incorporating a microcapsule according to any one of clauses 1 to 26, or a composition according to any one of clauses 27 to 32.

## Claims

1. A microcapsule comprising:
(a) a hydrophilic phase comprising a water-soluble ingredient;
(b) a lipophilic phase; and
(c) a plant-based protein hydrogel shell.

2. A microcapsule according to claim 1, wherein the hydrophilic phase comprises water.

3. A microcapsule according to claim 1 or claim 2, wherein the water-soluble ingredient is selected from one or more of Vitamin B1, Vitamin B2, Vitamin B3, Vitamin B5, Vitamin B6, Vitamin B7, Vitamin B9, Vitamin B12, Vitamin C, panthenol, α-hydroxy acids, water-soluble minerals salts, water-soluble plant extracts and yeasts, enzymes, antibiotics, oligopeptides, proteins and protein hydrolysates.

4. A microcapsule according to any preceding claim, wherein the plant-based protein(s) in the plant-based protein hydrogel shell is obtained from soybean, pea, rice, potato, wheat, corn zein or sorghum; preferably the plant protein(s) is selected from soy protein, pea protein, potato protein, rapeseed protein and/or rice protein.

5. A microcapsule according to any preceding claim, wherein the plant-based protein hydrogel shell is a self-assembled plant-based protein hydrogel shell.

6. A microcapsule according to any preceding claim, wherein the plant-based protein hydrogel shell comprises plant-based proteins having a protein secondary structure with at least 40% intermolecular β-sheet, at least 50% intermolecular β-sheet, at least 60% intermolecular β-sheet, at least 70% intermolecular β-sheet, at least 80% intermolecular β-sheet, or at least 90% intermolecular β-sheet;
and/or
wherein the plant-based protein hydrogel shell has a storage modulus (G') at 10rad/s of greater than 500Pa, greater than 1000Pa, greater than 2500Pa, greater than 3000Pa, greater than 4000Pa.

7. A microcapsule according to any preceding claim, wherein the plant-based protein hydrogel shell comprises protein aggregates with a median average length of between 50 to 500nm or a mean average length of between 50 to 500nm; or 80% of the aggregates have an average length of between 50 to 500nm;
and/or the aggregates may have a median height of between 5 to 50nm; or the aggregates may have a mean average height of between 5 to 50nm; or 80% of the aggregates have an average height of between 5 to 50nm;
preferably, the aggregates have a median average length of between 50 to 500nm and/or a median average height of between 5 to 50nm.

8. A microcapsule according to any preceding claim, wherein the hydrophilic phase is dispersed in the lipophilic phase.

9. A microcapsule according to any preceding claim, wherein the hydrophilic phase and the lipophilic phase are encapsulated by the plant-based protein hydrogel shell.

10. A microcapsule according to any preceding claim, wherein the plant-based protein hydrogel shell has a thickness in the range 10 nm to 50,000 µm, preferably in the range 10 µm to 100 µm.

11. A method for preparing a microcapsule according to any preceding claim, comprising:
(a) emulsifying a hydrophilic phase comprising a water-soluble ingredient in a first lipophilic phase to give a primary emulsion;
(b) re-emulsifying said primary emulsion in a plant-based protein solution comprising one or more plant-based protein(s), wherein said plant-based protein solution is at a temperature above the sol-gel transition temperature of the plant-based protein solution, to give a double emulsion;
(c) re-emulsifying said double emulsion in a second lipophilic phase to give a triple emulsion;
(d) inducing the plant-based protein(s) in the solution to undergo a sol-gel transition to form a plant-based protein hydrogel shell, wherein said plant-based protein hydrogel shell encapsulates said primary emulsion to form a microcapsule which is suspended in an external phase which is the second lipophilic phase; and
(e) washing the microcapsule to remove the second lipophilic phase.

12. A method according to claim 11, wherein the plant-based protein solution comprises one or more plant-based protein(s) in a solvent system, wherein the solvent system comprises miscible co-solvents; wherein a first co-solvent increases solubility of the plant-based protein(s), and a second co-solvent decreases solubility of the plant-based protein(s).

13. A method according to claim 12, wherein the first co-solvent is an organic acid; preferably acetic acid and/or an α-hydroxy acid; wherein the α-hydroxy acid may preferably be selected from glycolic acid, lactic acid, malic acid, citric acid and/or tartaric acid; with particularly preferred organic acids being acetic acid and/or lactic acid.

14. A method according to claim 12 or 13, wherein the second co-solvent(s) is an aqueous buffer solution, preferably selected from water, ethanol, methanol, acetone, acetonitrile, dimethylsulfoxide, dimethylformamide, formamide, 2-propanol, 1-butanol, 1-propanol, hexanol, t-butanol, ethyl acetate, hexafluoroisopropanol, more preferably water and/or ethanol, particularly preferably water.

15. The method according to any one of claims 11 to 14, wherein in step (d) the protein solution is heated to a first temperature above the sol-gel temperature of the one or more plant-based protein(s), then reduced to a second temperature below the sol-gel temperature of the one or more plant-based protein(s) to form the plant-based protein hydrogel shell.
